# EUROPEAN PATENT SPECIFICATION

(11) **EP 2 393 518 B1**
(45) Date of publication and mention of the grant of the patent: **04.08.2021**
(21) Application number: 10703808.5
(22) Date of filing: 04.02.2010
(51) Int. Cl.: G01N 33/531, C12N 15/10, C40B 40/08, C40B 50/06

(54) **STRUCTURED POLYCYCLIC PEPTIDE**
STRUKTURIERTE POLYCYCLISCHE PEPTIDE
PEPTIDE POLYCYCLIQUE STRUCTURÉ

(30) Priority: 04.02.2009 WO PCT/GB2009/000301; 06.08.2009 GB 0913775
(43) Date of publication of application: 14.12.2011
(60) Divisional application: 20208496.8
(73) Proprietor: BicycleRD Limited, Cambridge CB22 3AT (GB)
(72) Inventor: HEINIS, Christian, CH-1015 Lausanne (CH); TOUATI, Jeremy, CH-1004 Lausanne (CH); WINTER, Gregory, Paul, Cambridge CB2 2QH (GB)
(74) Representative: Maschio, Antonio
(86) International application number: PCT/EP2010/000691
(87) International publication number: WO 2010/089117

(56) References cited:
- WO-A2-2009/098450
- HEINIS CHRISTIAN ET AL: "Phage-encoded combinatorial chemical libraries based on bicyclic peptides" NATURE CHEMICAL BIOLOGY, vol. 5, no. 7, July 2009 (2009-07), pages 502-507, XP007913181 cited in the application
- CABRAS TIZIANA ET AL: "HPLC-MS characterization of cyclo-statherin Q-37, a specific cyclizatin product of human salivary statherin generated by transglutaminase 2" JOURNAL OF SEPARATION SCIENCE, vol. 29, no. 17, November 2006 (2006-11), pages 2600-2608, XP7913191 cited in the application
- KEMP DS & MCNAMARA P: "Peptides containing .beta.-turns I-- cyclo-(gly-1-cys-gly)3 triply bridged by 1,3,5-tris-(thiomethyl)benzene", TETRAHEDRON LETTERS, vol. 22, no. 46, 23 March 1981 (1981-03-23), pages 4571-4574,
- TIMMERMAN P ET AL: "A Combinatorial Approach for the Design of Complementary-determining Region-derived Peptidomimetics with in Vitro Anti-tumoral Activity", THE JOURNAL OF BIOLOGICAL CHEMISTRY, vol. 284, no. 49, 6 October 2009 (2009-10-06), pages 34126-34134,

## Description

The invention relates to peptide ligands whose structure is constrained by binding to a compound which provides a structural backbone, imparting a conformation to the peptide ligands. In particular, the invention relates to peptides which adopt a polycyclic conformation by end-to-end cyclisation and binding to a multidentate structural core.

### Introduction

The generation of molecules with high affinity and specificity for biological targets is a central problem in chemistry, biology and pharmaceutical sciences. In particular, binding ligands are important for the creation of drugs that can intervene with biological processes. The creation of ligands that bind to a chosen target ligand usually involves a process of generating a plurality of putative binding molecules and testing said molecules for their binding properties.

Polypeptides tethered to a synthetic molecular structure are known in the art (Kemp, D. S. and McNamara, P. E., J. Org. Chem, 1985; Timmerman, P. et al., ChemBioChem, 2005). Meloen and co-workers had used tris(bromomethyl)benzene and related molecules for rapid and quantitative cyclisation of multiple peptide loops onto synthetic scaffolds for structural mimicry of protein surfaces (Timmerman, P. et al., ChemBioChem, 2005). Methods for the generation of candidate drug compounds wherein said compounds are generated by linking cysteine containing polypeptides to a molecular scaffold as for example tris(bromomethyl)benzene are disclosed in WO 2004/077062 and WO 2006/078161.

WO2004/077062 discloses a method of selecting a candidate drug compound. In particular, this document discloses various scaffold molecules comprising first and second reactive groups, and contacting said scaffold with a further molecule to form at least two linkages between the scaffold and the further molecule in a coupling reaction.

WO2006/078161 discloses binding compounds, immunogenic compounds and peptidomimetics. This document discloses the artificial synthesis of various collections of peptides taken from existing proteins. These peptides are then combined with a constant synthetic peptide having some amino acid changes introduced in order to produce combinatorial libraries. By introducing this diversity via the chemical linkage to separate peptides featuring various amino acid changes, an increased opportunity to find the desired binding activity is provided. Figure 7 of this document shows a schematic representation of the synthesis of various loop peptide constructs. However, the peptides produced have single specificities. Where multiple peptide loops are provided, the loops cooperate to bind to a single target.

In our copending international patent application WO2009098450 we disclose the use of biological selection technology, such as phage display, to select peptide ligands tethered to synthetic molecular structures. These peptide ligands show specificity for target molecules. WO2004/077062 describes peptide ligands having a variety of loops subtended between attachment points on a molecular scaffold. Although this document discusses peptide cyclisation, it uses this term to define peptides bound to a scaffold, whether circular or not. In general, in the prior art a linear peptide can be subtended between (n+1) attachment points to generate n loops.

Synthetic cyclic peptides are typically produced by connecting the ends of side chain protected peptides which allows the use of a wide range of ligation chemistries. In some cases, the linear peptide precursors are produced by recombinant expression as for example polypeptides with long chains (> 50 amino acids) that are difficult to synthesize chemically or combinatorial peptide libraries that are generated by phage display, ribosome display, yeast display or other display techniques. The cyclisation of such unprotected peptides is more challenging and requires ligation strategies and reagents that are orthogonal to the peptides.

The present inventors show herein that the formation of multicyclic ligands, that is ligands comprising n loops subtended between n attachment points on a molecular scaffold, can take place if the peptide is cyclised by joining the N and C termini, forming n loops.

### Summary of the Invention

The disclosure provides a peptide ligand comprising a polypeptide linked to a molecular scaffold at n attachment points, wherein said polypeptide is cyclised and forms n separate loops subtended between said n attachment points on the molecular scaffold, wherein n is greater than or equal to 2.

The polypeptide is preferably cyclised by N- to C-terminal fusion, and can be cyclised before or after attachment to the molecular scaffold. Attachment before cyclisation is preferred.

Several methods are known in the art for peptide cyclisation. For example, the polypeptide is cyclised by N-C crosslinking, using a crosslinking agent such as EDC.

In another aspect, the peptide can be designed to comprise a protected N^{α} or C^{α} derivatised amino acid, and cyclised by deprotection of the protected N^{α} or C^{α} derivatised amino acid to couple said amino acid to the opposite terminus of the polypeptide.

In a preferred aspect, the polypeptide is cyclised by enzymatic means. For example, the enzyme is a transglutaminase, for instance a microbial transglutaminase, such as *Streptomyces mobaraensis* transglutaminase. In order to take advantage of enzymatic cyclisation, it may be necessary to incorporate an N- and/or C-terminal substrate sequence for the enzyme in the polypeptide. Some or all of the substrate sequence(s) can be eliminated during the enzymatic reaction, meaning that the cyclised polypeptide may not comprise the substrate sequences in its final configuration.

In one aspect, the peptide ligand is multispecific, and is capable of binding to more than one separate target. In a preferred embodiment the loop formed by polypeptide cyclisation binds to a target which is different to that bound by at least one other loop. This confers a number of advantages, in that cyclisation can be performed at any time when it is desired to impart the peptide ligand with the ability to bind another target.

If the peptide ligand is constructed by assembly of different loops of known specificity into one polypeptide, multispecificity can be determined by the number of loops. For example if the peptide ligand comprises three loops when cyclised, it can be mono-, bi- or tri-specific. The targets may be entirely different molecular entities, different epitopes on the same molecule, or multiple copies of the same molecule.

The value of n can be 2 or more, and is advantageously 3. Peptide ligands with higher numbers of loops are envisaged, for example 4, 5, 6, 7, 8 or more.

The invention provides a repertoire of peptide ligands as described in the appended set of claims.

According to the invention, the repertoire is displayed using a genetic display system. Applicable systems include phage display, bacterial display, yeast display, ribosome or polysome display, mRNA display and in vitro expression in artificial microcapsules. An mRNA display system is described in Litovchick et al., PNAS October 7, 2008 vol. 105 no. 40; 15293-15298. The preferred technique is phage display using a filamentous bacteriophage.

The repertoire may be naive, or may have been selected for binding to one or more targets. Selection for binding to the one or more targets can be carried out before cyclisation, after cyclisation, or both.

### Brief Description of the Figures

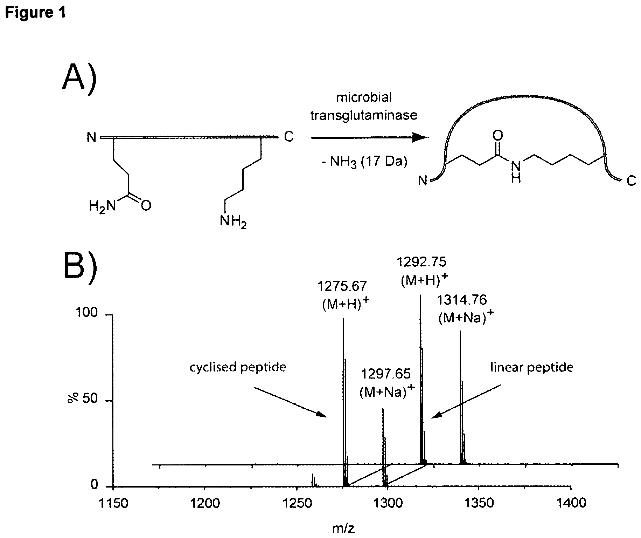

**Figure 1****.** Cyclisation of linear peptides with microbial transglutaminase (MTGase). (A) Schematic representation of the enzyme catalysed reaction. The side chains of the glutamine and lysine residues of a linear peptide are ligated by the MTGase to yield a cyclic peptide with a stable amide bond. (B) Mass spectra of peptide 1 (H-WALQRPHGGGKS-NH₂; the glutamine and lysine residues that participate in the reaction are underlined) before and after incubation with MTGase.

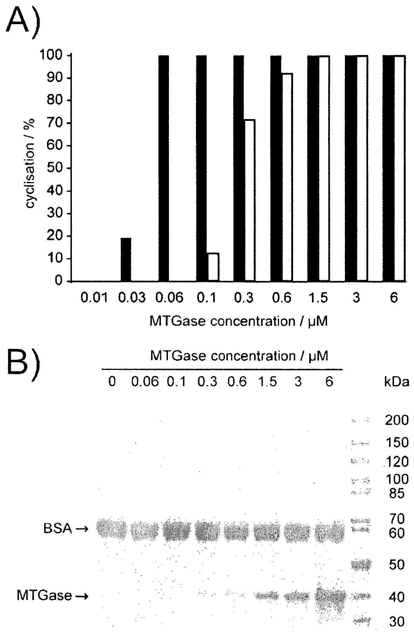

**Figure 2****.** Activity and substrate specificity of MTGase. (A) Peptide 3 (black) and 4 (white) were incubated with different MTGase concentrations. The cyclisation efficiency indicated as % of cyclic peptide was determined by LC/MS. (B) SDS-PAGE of bovine serum albumin (BSA; 5 µg) treated with different MTGase concentrations. No cross-linkage of BSA through linkage of surface-exposed glutamine and lysine residues was observed.

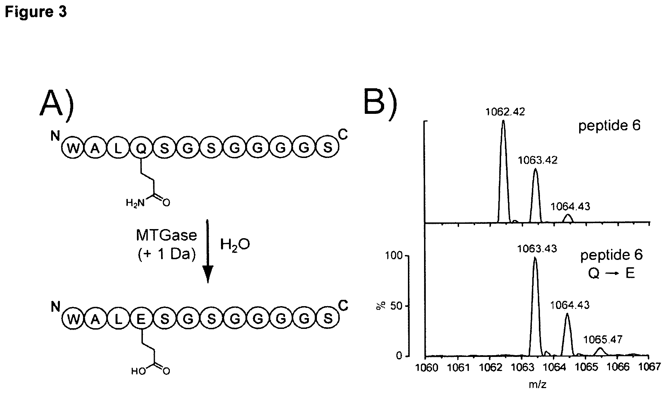

**Figure 3****.** Deamidation reaction. (A) Schematic representation of the transformation of glutamine into glutamate residue in peptide 6 by MTGase. (B) Mass spectra of peptide 6 before and after incubation with MTGase. Singly charged species, [M+H]⁺, are shown in each spectrum.

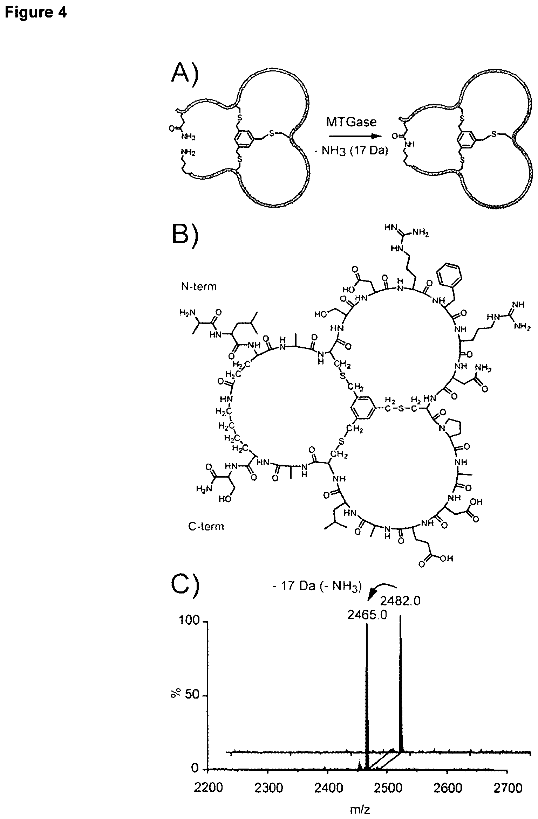

**Figure 4****.** Generation of tricyclic peptides. (A) Schematic representation of the transglutamination reaction linking the terminal appendices of a bicyclic peptide. (B) Chemical structure of a tricyclic peptide obtained by subsequently reacting peptide 7 with tris-(bromomethyl)benzene (TBMB) and treatment with MTGase. (C) Mass spectra of the peptide 7-TBMB conjugate before and after treatment with MTGase.

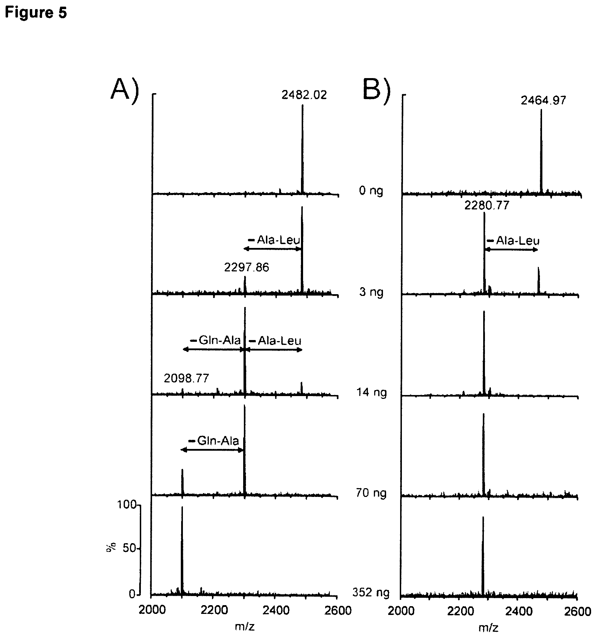

**Figure 5****.** Susceptibility of bi- and tricyclic peptides to aminopeptidase cleavage. Mass spectrometric analysis of bicyclic (A) and tricyclic (B) peptide 7 treated with different amounts of leucyl aminopeptidase from *Aeromonas Proteolytica.* The mass differences correspond to the loss of exocyclic amino acids present at the N-terminus of the peptide. The quantity of aminopeptidase added to the reactions (in ng) is indicated.

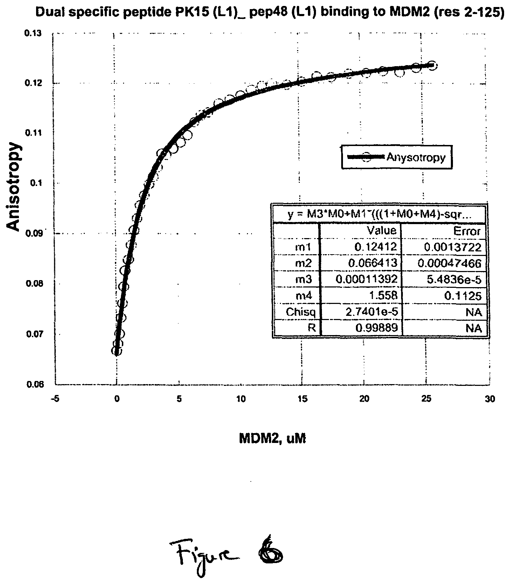

**Figure 6** shows a fluorescence anisotropy plot for a dual specific peptide comprising loops from PEP48 and PK15.

### Detailed Description of the Invention

Unless defined otherwise, all technical and scientific terms used herein have the same meaning as commonly understood by those of ordinary skill in the art, such as in the arts of peptide chemistry, cell culture and phage display, nucleic acid chemistry and biochemistry. Standard techniques are used for molecular biology, genetic and biochemical methods (see Sambrook et al., Molecular Cloning: A Laboratory Manual, 3rd ed., 2001, Cold Spring Harbor Laboratory Press, Cold Spring Harbor, NY; Ausubel et al., Short Protocols in Molecular Biology (1999) 4th ed., John Wiley & Sons, Inc.).

A peptide ligand, as referred to herein, refers to a peptide covalently bound to a molecular scaffold. Typically, such peptides comprise two or more reactive groups which are capable of forming covalent bonds to the scaffold, and a sequence subtended between said reactive groups which is referred to as the loop sequence, since it forms a loop when the peptide is bound to the scaffold.

The reactive groups are groups capable of forming a covalent bond with the molecular scaffold. Typically, the reactive groups are present on amino acid side chains on the peptide. Preferred are amino-containing groups such as cysteine, lysine and selenocysteine.

Multispecificity is the ability to bind to two or more targets. Typically, binding peptides are capable of binding to a single target, such as an epitope in the case of an antibody, due to their conformational properties. However, peptides can be developed which can bind to two or more targets; dual specific antibodies, for example, as known in the art as referred to above. In the present invention, the peptide ligands are capable of binding to two or more targets and are therefore multispecific. Preferably, they bind to two targets, and are dual specific. The binding may be independent, which would mean that the binding sites for the targets on the peptide are not structurally hindered by the binding of one or other of the targets. In this case both targets can be bound independently. More generally it is expected that the binding of one target will at least partially impede the binding of the other. Multispecific peptides can be formed by joining together individual loops of peptide ligands which bind to individual targets. The loops which are joined together may be adjacent loops, or may be separated by a third loop, or even further loops. Where the loops are placed directly adjacent in the multispecific peptide, it is preferred that one of the reactive groups defining one of the loops is omitted, to avoid effective duplication of reactive groups at one position.

A target is a molecule or part thereof to which the peptide ligands bind. Typically, the target will be analogous to an epitope, and thus may take the form of different epitopes on the same molecule, or different epitopes on different molecules. Where the targets are on the same molecule, the use of a dual specific ligand will increase the avidity of the ligand for the molecule, and may impart other properties due to cross-linking the molecule or the occupation of defined functional parts of the molecule.

The molecular scaffold is any molecule which is able to connect the peptide at multiple points to impart one or more structural features to the peptide. It is not a cross-linker, in that it does not merely replace a disulfide bond; instead, it provides two or more attachment points for the peptide. Preferably, the molecular scaffold comprises at least three attachment points for the peptide, referred to as scaffold reactive groups. These groups are capable of reacting to the reactive groups on the peptide to form a covalent bond. Preferred structures for molecular scaffolds are described below.

A repertoire is a collection of variants, in this case polypeptide variants, which differ in their sequence. Typically, the location and nature of the reactive groups will not vary, but the sequences forming the loops between them can be randomised. Repertoires differ in size, but should be considered to comprise at least 10² members. Repertoires of 10¹¹ or more members can be constructed.

Screening for binding activity (or any other desired activity) is conducted according to methods well known in the art, for instance from phage display technology. For example, targets immobilised to a solid phase can be used to identify and isolate binding members of a repertoire. Screening allows selection of members of a repertoire according to desired characteristics.

The term library refers to a mixture of heterogeneous polypeptides or nucleic acids. The library is composed of members, each of which has a single polypeptide or nucleic acid sequence. To this extent, library is synonymous with repertoire. Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members.

Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

Preferably, a library of nucleic acids encodes a repertoire of polypeptides. Each nucleic acid member of the library preferably has a sequence related to one or more other members of the library. By related sequence is meant an amino acid sequence having at least 50% identity, suitably at least 60% identity, suitably at least 70% identity, suitably at least 80% identity, suitably at least 90% identity, suitably at least 95% identity, suitably at least 98% identity, suitably at least 99% identity. Identity is suitably judged across a contiguous segment of at least 3 amino acids, suitably at least 4, 5, 6, 7, 8, 9 or 10 amino acids, suitably least 12 amino acids, suitably least 14 amino acids, suitably least 16 amino acids, suitably least 17 amino acids or the full length of the reference sequence.

A functional group, attached to a peptide ligand, is a group which, for example, mediates a further binding activity or permits the binding of an effector group. Thus, functional groups include antibodies and binding fragments thereof, further peptide ligands as described herein, chemical reactive groups, and the like.

An effector group is a group attached to the peptide ligand which has a specific activity. For instance, it may be a protein which increases the half life of the peptide ligand, such as human serum albumin (HSA). Effector groups also include drugs, such as cytotoxic drugs, immunoeffectors, such as antibody Fc regions, and compounds which conform to the following parameters: not more than 5 hydrogen bond donors (nitrogen or oxygen atoms with one or more hydrogen atoms); not more than 10 hydrogen bond acceptors (nitrogen or oxygen atoms); a molecular weight under 500 daltons; and an octanol-water partition coefficient log P of less than 5.

### A. Peptide ligands

The design and production of peptide ligands is described in our published International patent application WO 2009/098450, as well as International patent applications WO 2004/077062 and WO 2006/078161. The following aspects summarise constructing peptide ligands.

### (i) Molecular scaffold

The molecular scaffold is sometimes referred to as the 'molecular core' or 'connector compound'. Suitably, the molecular scaffold possesses molecular symmetry. Suitably, the molecular scaffold possesses three scaffold reactive groups and possesses threefold symmetry. This has the advantage of producing only a single reaction product. If the molecular scaffold is not a symmetric molecule, then multiple reaction products can be produced. This can lead to complications, or require that the desired isomer be separated from the other reaction products.

Suitably the molecular scaffold may be a small molecule. Suitably the molecular scaffold is a small organic molecule.

Suitably the molecular scaffold may be, or may be based on, natural monomers such as nucleosides, sugars, or steroids. Suitably the molecular scaffold may comprise a short polymer of such entities, such as a dimer or a trimer.

Suitably the molecular scaffold may comprise or may consist of tris(bromomethyl)benzene, especially 1,3,5-Tris(bromomethyl)benzene ('TBMB'), or a derivative thereof.

Another suitable molecular scaffold is 2,4,6-Tris(bromomethyl)mesitylene. It is similar to 1,3,5-Tris(bromomethyl)benzene but contains additionally three methyl groups attached to the benzene ring. This has the advantage that the additional methyl groups may form further contacts with the polypeptide and hence add additional structural constraint.

The molecular scaffold of the present invention is selected from either a small molecule or a macromolecular structure. The said molecular scaffold is composed of organic, inorganic or organic and inorganic components.

In a preferred embodiment, the molecular scaffold is a small organic molecule as for example a linear alkane. More suitably the molecular scaffold is a branched alkane, a cyclic alkane, a polycyclic alkane, an aromate, a heterocyclic alkane or a heterocyclic aromate, which offer the advantage of being less flexible (i.e. more rigid). Most suitably the molecular scaffold comprises a benzylic group.

In another embodiment, the molecular scaffold is selected from a macromolecular structure as for example a polypeptide, a polynucleotide or a polysaccharide.

The molecular scaffold of the invention contains chemical groups that allow functional groups of the polypeptide of the encoded library of the invention to form covalent links with the molecular scaffold. Said chemical groups are selected from a wide range of functionalities including amines, thiols, alcohols, ketones, aldehydes, nitriles, carboxylic acids, esters, alkenes, alkynes, anhydrides, succinimides, maleimides, azides, alkyl halides and acyl halides.

### (ii) Polypeptide

The reactive groups of the encoded polypeptides are suitably provided by side chains of natural or non-natural amino acids. The reactive groups of the encoded polypeptides are suitably selected from thiol groups, amino groups, carboxyl groups, guanidinium groups, phenolic groups or hydroxyl groups. The reactive groups of the encoded polypeptides may suitably be selected from azide, keto-carbonyl, alkyne, vinyl, or aryl halide groups. The reactive groups of the encoded polypeptides for linking to a molecular scaffold may suitably be the amino or carboxy termini of the polypeptide.

In some embodiments each of the reactive groups of the polypeptide for linking to a molecular scaffold are of the same type. For example, each reactive group may be a cysteine residue.

Suitable amino acids of the members of the genetically encoded combinatorial chemical libraries can be replaced by any natural or non-natural amino acid. Excluded from these exchangeable amino acids are the ones harbouring functional groups for cross-linking the polypeptides to a molecular core. A group of adjacent amino acids that can be varied is defined as a polypeptide segment. The size of a single polypeptide segment suitably ranges from 1 to 20 amino acids. The polypeptide segments have either random sequences, constant sequences or sequences with random and constant amino acids. The amino acids with reactive groups are either located in defined or random positions within the encoded polypeptide of the invention.

In one embodiment, the polypeptide segments that are bounded by two amino acids harbouring reactive groups for bonding with a molecular scaffold/molecular core are short amino acid sequences of 10 or fewer amino acids. Reaction of said encoded polypeptide sequences with a molecular core generates library members with high conformational constraint. Conformational constrained ligands are generally more specific and have higher binding affinities.

### (iii) Reactive groups of the polypeptide

The molecular scaffold of the invention may be bonded to the polypeptide via functional or reactive groups on the polypeptide. These are typically formed from the side chains of particular amino acids found in the polypeptide polymer. Such reactive groups may be a cysteine side chain, a lysine side chain, or an N-terminal amine group or any other suitable reactive group.

Suitably at least one reactive group is a cysteine group. Groups such as lysine or the N-terminal amines are typically not reactive enough to bond with the molecular scaffold on their own within a convenient time frame. However, once the molecular scaffold has been attracted or bonded to at least one cysteine, then ordinary reaction kinetics mean that the lysine or amine bonds can rapidly and stably form thereafter. For this reason, suitably at least one of the reactive groups is a cysteine group.

If reactive groups on the polypeptide other than cysteine/lysine/amine groups are desired, then a different molecular scaffold may be chosen in order to pair with the particular functional reactive groups of choice on the target polypeptide.

Suitably cysteine, lysine or amine groups are used as the functional or reactive groups on the polypeptide of interest.

Suitably at least three covalent bonds are formed between the molecular scaffold and the polypeptide of interest.

In some embodiments, four bonds or even more may be formed between the molecular scaffold and the polypeptide of interest. However, if more than four bonds are used, then typically the product mixtures formed become increasingly complex and may hinder the subsequent uses or applications. For this reason, three bonds or four bonds between the molecular scaffold and the polypeptide of interest are preferred.

Suitable reactive groups of natural amino acids are the thiol group of cysteine, the amino group of lysine, the carboxyl group of aspartate or glutamate, the guanidinium group of arginine, the phenolic group of tyrosine or the hydroxyl group of serine. Non-natural amino acids can provide a wide range of reactive groups including an azide, a keto-carbonyl, an alkyne, a vinyl, or an aryl halide group. The amino and carboxyl group of the termini of the polypeptide can also serve as reactive groups to form covalent bonds to a molecular scaffold/molecular core.

The encoded polypeptides of the invention suitably contain at least three reactive groups. Said polypeptides can also contain four or more reactive groups. The more reactive groups are used, the more diversity segments can be tethered to the molecular scaffold/molecular core. However, the linkage of excessive numbers of reactive groups to a molecular scaffold/molecular core is not recommended since this can lead to an unmanageable number of product isomers. Suitably three, four or five covalent bonds to a molecular scaffold are used; most suitably three or four covalent bonds; most suitably three covalent bonds.

### B: Attachment of Scaffolds to Phage peptides

Detailed conditions for the attachment of scaffolds to phage peptides without destroying phage infectivity are described in our international patent application WO 2009/098450. The attachment of scaffold molecules involves the following principles.

In particular, the reduction of the cysteines in the target polypeptide is required for the most efficient reaction. Subsequently, the reducing agent used to chemically reduce those cysteines is usually removed in order to perform the desired attachment. Thiol groups of a phage encoded polypeptide may be reduced with reducing agent prior to molecular scaffold attachment. In such embodiments, in particular in phage display embodiments, or in particular when the reducing agent is TCEP, the excess of reducing agent is suitably removed by filtration e.g. filtration of the phage.

However in some embodiments, the reducing agent may be retained in the reaction, for example at a level of 30µM TCEP.

Re-oxidation of the thiol groups after removal of TCEP is suitably prevented by degassing of the reaction buffer.

Re-oxidation of the thiol groups is also suitably prevented by complex formation of metal ions by chelation, for example chelation with ethylenediaminetetraacetic acid (EDTA).

Most suitably re-oxidation of the thiol groups is prevented or inhibited by both chelation and use of degassed buffers.

In one embodiment of the present invention, attachment of the polypeptide to the molecular scaffold is accomplished by reacting the reactive groups of the polypeptide such as thiol groups of a phage encoded polypeptide with the molecular scaffold for one hour.

Suitably they are reacted at 30°C.

Suitably they are reacted with molecular scaffold (such as tris(bromomethyl)benzene) at a concentration of 10 µM to 40µM.

Suitably reaction is in aqueous buffer.

Suitably reaction is at pH 8.

Suitably reaction buffer contains acetonitrile. Suitably reaction buffer contains 20% acetonitrile.

These reaction conditions are optimized to quantitatively react thiol groups of a polypeptide with the reactive groups of tris(bromomethyl)benzene. Under the same reaction conditions, about 20% of the phage particles remain infective to bring the genetic code into bacterial cells for propagation and decoding.

In one embodiment the molecular scaffold, such as TBMB, may be attached to the target polypeptide, such as a phage encoded polypeptide, by reaction (incubation) of thiol groups of the polypeptide for one hour at 30°C with 10 µM TBMB (i.e. tris(bromomethyl)benzene) in the presence of 10 µM TCEP in aqueous buffer pH 8 containing 20% acetonitrile. In another embodiment, the reaction can be carried out using 40 µM TBMB in the presence of 30 µM TCEP in the same buffer.

### C: Polypeptide Cyclisation

### Chemical cyclisation

The invention also relates to peptide ligands having two or more loops, in which the polypeptide in the ligand is cyclised in order to create one loop. For example, tricyclic polypeptides joined to a molecular scaffold can be created by joining the N- and C- termini of a bicyclic polypeptide joined to a molecular scaffold according to the present invention. In this manner, the joined N and C termini create a third loop, making a tricyclic polypeptide. This embodiment is suitably not carried out on phage, but is suitably carried out on a peptide ligand, that is a polypeptide-molecular scaffold conjugate, of the invention. Joining the N- and C- termini is a matter of routine peptide chemistry. In case any guidance is needed, the C-terminus may be activated and/or the N- and C- termini may be extended for example to add a cysteine to each end and then join them by disulphide bonding. Alternatively the joining may be accomplished by use of a linker region incorporated into the N/C termini. Alternatively the N and C termini may be joined by a conventional peptide bond. Alternatively any other suitable means for joining the N and C termini may be employed, for example N-C-cyclisation could be done by standard techniques, for example as disclosed in Linde et al. Peptide Science 90, 671-682 (2008) "Structure-activity relationship and metabolic stability studies of backbone cyclisation and N-methylation of melanocortin peptides", or as in Hess et al. J. Med. Chem. 51, 1026-1034 (2008) " backbone cyclic peptidomimetic melanocortin-4 receptor agonist as a novel orally administered drug lead for treating obesity".

In general, the procedures used to construct cyclised peptides from their linear molecules rely on the known principles of peptide synthesis; most conveniently, the procedures can be performed according to the known principles of solid phase peptide synthesis. During solid phase synthesis of a cyclised polypeptide, a protected linkage group is coupled to the N-terminus of the peptide chain or to the peptide resin in a similar procedure to the coupling of other amino acids. After completion of the polypeptide assembly, the protective group is removed from the linkage group's functional group and the cyclisation is accomplished by coupling the linkage group's functional group and a second functional group selected from a second linkage group, a side chain of an amino acid residue of the peptide sequence, and an N-terminal amino acid residue.

The linkage group can be an N^{α} or C^{α} derivatised amino acid, and preferably comprises one nitrogen atom of the peptide backbone connected to a chemical linker comprising an amide, thioether, thioester, disulfide, urea, carbamate, or sulfonamide, wherein at least one linkage group is connected via said bridging group to form a cyclic structure with a moiety selected from the group consisting of a second linkage group, the side chain of an amino acid residue of the sequence or a terminal amino acid residue.

The methodology for producing the linkage groups is described in international patent applications published as WO 95/33765 and WO 98/04583 and in U.S. Pat. Nos. 5,770,687 and 5,883,293.

Methods for design and synthesis of cyclised plypeptides are disclosed in U.S. Pat. Nos. 5,811,392; 5,874,529; 5,883,293; 6,051,554; 6,117,974; 6,265,375, 6,355,613, 6,407,059, 6,512,092 and international applications WO 95/33765; WO 97/09344; WO 98/04583; WO 99/31121; WO 99/65508; WO 00/02898; WO 00/65467 and WO 02/062819.

If the cyclisation is not carried out on phage, it will be noted that this third loop will not typically be available for selection (because it is not produced on the phage but only on the polypeptide-molecular scaffold conjugate) and so its use for other such biological functions still advantageously leaves both loops 1 and 2 for selection/creation of specificity. Thus the invention also relates to such tricyclic polypeptides and their manufacture and uses.

### Enzymatic cyclisation

As disclosed in the examples, amino acid sequences can be quantitatively cyclised with a microbial transglutaminase (see Figure 1A). In nature, transglutaminases (TGases) catalyse the acyl transfer reaction between the carboxyamide groups of glutamine in peptides and a variety of primary amines. (Beninati, S. & Piacentini, M. The transglutaminase family: an overview: minireview article. Amino Acids 26, 367-372 (2004)). The reaction, widely observed in plants, animals and microorganisms, leads to post-translational modification of proteins through either the formation of isopeptide bonds or the covalent attachment of amines. The ligation activity of TGases has been exploited in the food industry by cross-linking of proteins and by binding low-molecular weight compounds to carrier proteins using mammalian transglutaminase from guinea pig or microbial TGase (Yokoyama, K., Nio, N. & Kikuchi, Y. Properties and applications of microbial transglutaminase. Appl Microbiol Biotechnol 64, 447-454 (2004)). TGases have also been used for the labelling of proteins with small molecules tags as for example the attachment of biotin (Josten, A., Haalck, L., Spener, F. & Meusel, M. Use of microbial transglutaminase for the enzymatic biotinylation of antibodies. J Immunol Methods 240, 47-54 (2000)) or fluorescent probes (Mindt, T.L. et al. Modification of different IgG1 antibodies via glutamine and lysine using bacterial and human tissue transglutaminase. Bioconjug Chem 19, 271-278 (2008)) to antibodies for immunochemical applications. In other applications, TGases have been used for the cross-linking of proteins (Takazawa, T., Kamiya, N., Ueda, H. & Nagamune, T. Enzymatic labeling of a single chain variable fragment of an antibody with alkaline phosphatase by microbial transglutaminase. Biotechnol Bioeng 86, 399-404 (2004)). However, to our knowledge, the class of TGases has so far not been used for the cyclisation of peptides.

Although it has been found in human saliva that 1% of the natural peptide statherin is transformed by the action of transglutaminase 2 into a cyclic derivative (Cabras, T. et al. HPLC-MS characterization of cyclo-statherin Q-37, a specific cyclization product of human salivary statherin generated by transglutaminase 2. J Sep Sci 29, 2600-2608 (2006)), it was initially unclear whether peptides with varying amino acid sequences and lengths could be cyclised efficiently by TGases. As shown in the examples, microbial TGase can quantitatively cyclise peptides. The minimal substrate requirements are also described. Furthermore, we have used the enzymatic cyclisation strategy with bicyclic peptide ligands to generate tricyclic peptide structures.

### D: Targets

Peptide ligands according to the present invention may be designed to bind to any given target. One skilled in the art will appreciate that the choice of target molecule is large and varied. They may be for instance human or animal proteins, cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA78, Eotaxin, Eotaxin-2, Exodus-2, FGF-acidic, FGF-basic, fibroblast growth factor-10, FLT3 ligand, Fractalkine (CX3C), GDNF, G-CSF, GM-CSF, GF- I, insulin, IFNy, IGF-I, IGF-II, IL-la, IL-1 (3, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-17a, IL-17c,IL-17d, IL-17e, IL-17f, IL-18 (IGIF), IL-21, IL-22, IL-23, IL-31, IL-32, IL-33, IL-34, Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a. a.), MDC (69 a. a.), MCP-1 (MCAF), MCP-2, MCP-3, MCP-4, MDC (67 a. a.), MDC (69 a. a.), MIG, MIP-Ia, MIP-1p, MIP-3a, MIP3, MIP-4, myeloid progenitor inhibitor factor-1 (MPIF-1), NAP-2, Neurturin, Nerve growth factor, P-NGF, NT-3, NT-4, Oncostatin M, PDGF-AA, PDGF-AB, PDGF-BB, PF-4, RANTES, SDFIa, SDFIp, SCF, SCGF, stem cell factor (SCF), TARC, TGF-α, TGF-β, TGF- 2, TGF- 3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-y, HCC1,1-309, HER 1, HER 2, HER 3 and HER 4; Cytokine receptors include receptors for the foregoing cytokines. Chemokine targets include CC chemokine ligands CCL21/6Ckine, CCL12/MCP-5, CCL6/C10, CCL22/MDC, CCL14/HCC-1/HCC-3, CCL3L1/MIP-1 alpha Isoform LD78 beta, CCL23/Ck beta 8-1, CCL3/MIP-1 alpha, CCL28, CCL4L1/LAG-1, CCL27/CTACK, CCL4/MIP-1 beta, CCL24/Eotaxin-2/MPIF-2, CCL15/MIP-1 delta, CCL26-like/Eotaxin-3-like, CCL9/10/MIP-1 gamma, CCL26/Eotaxin-3, CCL19/MIP-3 beta, CCL11/Eotaxin,CCL20/MIP-3 alpha, CCL14a/HCC-1, CCL23/MPIF-1, CCL14b/HCC-3, CCL18/PARC, CCL16/HCC-4, CCL5/RANTES, CCL1/I-309/TCA-3, TAFA1/FAM19A1, MCK-2, TAFA5/FAM19A5, CCL2/JE/MCP-1, TAFA3/FAM19A3, CCL8/MCP-2, TAFA4/FAM19A4, CCL7/MCP-3/MARC, CCL17/TARC, CCL13/MCP-4 and CCL25/TECK; chemokine receptors include CCR1, CCR7, CCR2, CCR8, CCR3, CCR9, CCR4, CCR10, CCR5, CCRL2/LCCR/CRAM-A/B and CCR6; CXC chemokine ligands include CXCL13/BLC/BCA-1, CXCL10/IP-10/CRG-2, CXCL14/BRAK, LIX, CXCL16, CXCL15/Lungkine, CXCL5/ENA-78, CXCL9/MIG, CXCL6/GCP-2, CXCL7/NAP-2, CXCL1/2/3/GRO, CXCL4/PF4, CXCL1/GRO alpha/KC/CINC-1, CXCL12/SDF-1 alpha, CXCL2/GRO beta/MIP-2/CINC-3, CXCL12/SDF-1 beta, CXCL3/GRO gamma/CINC-2/DCIP-1, CXCL12/SDF-1, CXCL11/I-TAC, CXCL7/Thymus Chemokine-1 and CXCL8/IL-8; CXC chemokine receptors include CXCR3, CXCR7/RDC-1, CXCR4, CXCR1/IL-8 RA, CXCR5, CXCR2/IL-8 RB and CXCR6; TNF Superfamily ligands include 4-1BB Ligand/TNFSF9, LIGHT/TNFSF14, APRIL/TNFSF13, Lymphotoxin, BAFF/BLyS/TNFSF13B, Lymphotoxin beta/NFSF3, CD27 Ligand/TNFSF7, OX40 Ligand/TNFSF4, CD30 Ligand/TNFSF8, TL1A/TNFSF15, CD40 Ligand/TNFSF5, TNF-alpha/TNFSF1A, EDA (pan), TNF-beta/TNFSF1B, EDA-A1/Ectodysplasin A1, TRAIL/TNFSF10, EDA-A2, TRANCE/TNFSF11, Fas Ligand/TNFSF6, TWEAK/TNFSF12 and GITR Ligand/TNFSF18; TNF Superfamily receptors include 4-1BB/TNFRSF9/CD137, NGF R/TNFRSF16, BAFF R/TNFRSF13C, Osteoprotegerin/TNFRSF11B, BCMA/TNFRSF17, OX40/TNFRSF4, CD27/TNFRSF7, RANK/TNFRSF11A, CD30/TNFRSF8, RELT/TNFRSF19L, CD40/TNFRSF5, TACI/TNFRSF13B, DcR3/TNFRSF6B, TNFRH3/TNFRSF26, DcTRAIL R1/TNFRSF23, TNF RI/TNFRSF1A, DcTRAIL R2/TNFRSF22, TNF RII/TNFRSF1B, DR3/TNFRSF25, TRAIL R1/TNFRSF10A, DR6/TNFRSF21, TRAIL R2/TNFRSF10B, EDAR, TRAIL R3/TNFRSF10C, Fas/TNFRSF6/CD95, TRAIL R4/TNFRSF10D, GITR/TNFRSF18, TROY/TNFRSF19, HVEM/TNFRSF14, TWEAK R/TNFRSF12, Lymphotoxin beta R/TNFRSF3 and XEDAR; Toll-Like Receptors including TLR-1, TLR-2, TLR-3, TLR-4, TLR-5, TLR-6, TLR-7, TLR-8 and TLR-9; enzymes, including Cathepsin A, Cathepsin B, Cathepsin C, Cathepsin D, Cathepsin E, Cathepsin F, MMP 1, MMP2, MMP 3, MMP 7, MMP 8, MMP 9, MMP 10, MMP 11, MMP 12, MMP 13, MMP 14, MMP 15, MMP 16, MMP 17, MMP 19, MMP 20, MMP 21, MMP 23A, MMP 23B, MMP 26, MMP 27, MMP 28, urokinase, kallikreins, including KLK1, KLK2, KLK3, KLK4, KLK5, KLK6, KLK7, KLK8, KLK9, KLK10, KLK11, KLK12, KLK13, KLK14 and KLK15; components of the complement system; Intracellular signalling molecules and transcription factors; p53; and MDM2.

It will be appreciated that this list is by no means exhaustive.

Targets may also be large plasma proteins, such as serum albumins, as set forth below.

Where the polypeptide conjugate binds to two epitopes (on the same or different targets), the target molecules may be selected from this list. The targets may compete for binding to the polypeptide conjugate, such that they cannot both bind simultaneously. Alternatively, they may both bind simultaneously, such that the polypeptide conjugate bridges the targets.

### Example 1. Cyclic peptides binding to two or more targets

The work by Heinis et al., WO 2009/098450, demonstrates the isolation of a bicyclic peptide (PK15) against kallikrein (PK15). PK15 was made by a two stage process. A first bicyclic repertoire was created with diversity in both loops. After iterative selection with kallikrein, a set of consensus sequences emerged in the first loop, of which PK2 was representative. A second repertoire was then created, keeping the PK2 sequence in the first loop, and diversifying the second loop. After iterative selection with kallikrein, a set of consensus sequences emerged in the second loop. The two-stage process led to an improvement in binding affinity.

The emergence of consensus sequences in the first loop of PK2 suggests that this loop makes a significant contribution to binding. It does not exclude the possibility that the second loop also make a significant contribution to binding in PK2. Indeed the emergence of a second loop consensus sequence in PK15 suggests that the second loop does make a significant contribution to binding in PK15.

Nevertheless we wondered whether it might be possible to build a bicyclic peptide with binding specificities to two targets by combining individual loops from bicyclic peptides of different specificity. Although we expected to see a significant loss in binding affinity for each target, we considered that further mutagenesis should lead to variants with improved binding affinities. We also evaluated a tricyclic peptide made by fusion of the N and C termini of the bicyclic molecule.

Accordingly, we first synthesized a number of variants of the first loop of PK15 on the TBMB core in manner described earlier by Heinis et al. (2009), or variation as described below. For the tricyclic a further chemical step was taken to join the N-and C-terminus of a bicyclic peptide. The bicyclic peptides were purified by HPLC, checked by mass spectrometry, and dried by lyophilisation. The table summarises the IC50 for inhibition of kallikrein activity for each variant peptide.

For the other loop(s), in addition to sequences derived from the bicyclic kallikrein inhibitors, we also used sequences derived from bicyclic cathepsin G inhibitors (as also described by Heinis et al. 2009. Nature Chemical Biology 5, 502-507), and from bicyclic inhibitors against MDM2, an enzyme (an E3 ubiquitin ligase) that recognises the trans-activation domain of p53, the tumour suppressor, leading to ubiquitinylation and degradation of p53 by the proteosome. A nutlin inhibitor of the p53-MDM2 interaction can lead to *in vivo* activation of the p53 pathway, and it has been suggested that such agents may have potential as anti-cancer agents. We selected two bicyclic peptides (PEP10 and PEP48) against MDM2; the affinity of each synthetic peptide was micromolar or better. The PEP48 conjugate was shown by competition ELISA to bind to the same site as a linear peptide previously shown to block the p53-MDM2 interaction. The protocols for selection of these MDM2 binders, generally followed those described earlier in Heinis et al., 2009, Nature Chemical Biology 5, 502-507, but with some differences (mainly included the use of reducing agent during the selections and/or the inclusion of a protease), and the protocols are summarised in the Methods below.

The first loop of PK15 is underlined in each of the variants, and was combined with the first loop of the CG4 peptide (double underlined) directed against cathepsin G (Heinis et al, 1990), or the first loop of PEP48 (dashed underline) directed against MDM2 (see WO 2009/098450).

| **Peptide name** | **Peptide sequence** | IC50 |
|---|---|---|
| PK15L1-PK15L2 bicyclic | (H)-ACSDRFRNCPADEALCG-(OH) | 13 nM |
| X-CG4L1-PK15L1-Y bicyclic | (H)-ALCIFDLGFCSDRFRNCPADE-(OH) | 15 µM |
| CG4L1-PK15L1-PK15L2 tricyclic | (H)-ALCIFDLGFCSDRFRNCPADE-(OH) | 15 µM |
| PK15L1-CG4L1 bicyclic | (H)-ACSDRFRNCIFDLGFCG-(OH) | 200 nM |
| PK15L1-PEP48L1 bicyclic | (H)-ACSDRFRNCVRFGWTCG-(OH) | 11 µM |

The results show that the first loop of PK15 is able to inhibit kallikrein activity when combined with loops from bicyclic (or a tricyclic) peptides directed against a second target. However the inhibitory activity is much less than when combined with its cognate loop, and varies according to the sequences and/or order of the non-cognate loops. In the case of the tricyclic peptide, the cyclization from the corresponding bicyclic does not seem to compromise its kallikrein binding activity.

We also obtained results for the binding of the first loop of PEP48 in combination with PK15 (Fig. 6). Thus the PK15L1-PEP48L1 bicyclic peptide has a binding affinity (Kd=1.55uM) for MDM2 only two or three fold lower than that for the entire PEP48L1-PEP48L2 bicyclic peptide (Kd = 500-800 nM). This demonstrates that it is possible to combine loops from two cyclic peptides of different target specificity, and thereby to create a cyclic peptide with the two target specificities.

It is possible to improve the binding affinities of these cyclic peptides, for example by (a) synthesizing a mutated DNA cassette encoding the peptides with "spiked" oligonucleotides, (b) displaying said mutant peptide repertoire on phage, and (c) subjecting said phage repertoire to rounds of selection under increasingly stringent conditions (for example using lower concentrations of antigen, or more extensive washes of the phage bound to target. Furthermore by selecting the repertoire against each of the targets in turn (with or without intervening rounds of bacterial growth, depending on phage yields), it should be possible to ensure that the selection pressure is maintained on both targets.

### Methods

### Synthesis of TBMB-peptide conjugates

Initial reactions were performed to mimic the conditions used during phage selection. Typically, 5mg of the purified peptide was dissolved in 1ml water and 0.8 ml 50mM NH₃HCO₃ added, followed by 40µl of TCEP. TBMB (3 equivalents based on weight of peptide) dissolved in MeCN was added to the reaction. The reaction was left for 1.5 hrs then monitored by HPLC. On completion the reaction was purified by HPLC. Typically 0.5 to 1.5 mg of final product was obtained. This method gives rise to many by-products, the major product being the desired mass + 250 amu. This corresponds to addition of TCEP to the desired product, and that the yield of this product increases with reaction time. In addition other higher mass products corresponding to addition of a second TBMB were observed by MALDI-TOF mass spec, but were not isolated.

Based on the formation of TCEP adducts a preferred method was developed. Following cleavage of the peptide from the resin, it was either purified directly by HPLC or pre-treated with TCEP for 15 mins prior to HPLC purification. The product from the HPLC reaction, in the HPLC elution buffer (typically 6ml) is neutralised with 50mM NH₃HCO₃ (4ml) and TBMB added in MeCN as above. The addition of 10% THF results in a clear solution and therefore accelerates the reaction. Reactions are monitored by mass spec, but typically are complete in 1-2 hrs. There are minimal by-products from this reaction (though the presence of product +16 is observed by mass spec). The reaction requires concentration to remove organic solvents prior to HPLC purification otherwise the product tends to elute with the solvent front. Yields of product from this method are typically 0.5 to 1.5 mg from 3mg peptide, but this has not been optimised.

### Synthesis of tricyclic peptide

The tricyclic peptide CG4L1-PK15L1-PK15-L2 was synthesised as follows: approximately 1mg of the bicyclic X-CG4L1-PK15L1-Y (where X and Y represent portions of PK15L2) was dissolved in 2ml of 20mM NH₃HCO₃ and treated with EDC (0.8mg in 100µl water, 10 equivalents) and heated in microwave synthesiser at 50W starting at 0°C up to 37°C. Reaction progress was monitored at 15 mins and 30 mins when the cyclised product was the major product but a second loss of water was also observed. The reaction was purified by HPLC (semi-prep) to give the tricyclic conjugate as a single peak, yield 0.5mg.

### Kallikrein assays

Enzymes were purchased from Sigma Aldrich and substrates from Bachem AG. The assay buffer is composed of 10 mM Tris pH 7.4, 150 mM NaCl, 10 mM MgCI2, 1 mM CaCl₂, 0.1% BSA, 0.01% Triton X100 and 5% DMSO. Enzymes are incubated with inhibitors for 30 minutes at RT prior to addition of substrate. All experiments were recorded at 30°C for 90 minutes.

Assays were performed on a BMG Pherastar plate reader at wavelengths of exc/em 350/450 nm. Kallikrein was bought as a solution of 1080 µg/mL and diluted to a working concentration of 0.3 nM in assay buffer. Substrate Z-Phe-Arg-amc was solubilised at the stock concentration of 10 mM in DMSO and diluted to a working concentration of 300 µM with assay buffer. Inhibitors were solubilised in assay buffer to a stock concentration of 60 µM. 50 µL of each reagent is introduced in wells for a final volume of 150 µL per well. Final concentration of kallikrein in assay is 0.1 nM and substrate is 100 µM.

Final concentrations of inhibitors were : 0,5 nM, 1 nM, 2 nM, 5 nM, 8 nM, 10 nM, 20 nM, 50 nM, 80 nM, 100 nM, 200 nM, 500 nM, 800 nM, 1 µM, 2 µM, 5 µM, 8 µM, 10 µM and 20 µM. The initial rate of the reaction is obtained by plotting fluorescence = f (time) data and by fitting a linear trendline for each concentration of inhibitor. The inhibition curves are obtained by plotting initial rate = f ([I]) and IC₅₀ values can be evaluated.

### Phage production and purification

For the selections against MDM2, the phage peptide library with diversity of at least 4x10⁹ clones was prepared and TBMB conjugated as described earlier by Heinis et al., 2009 Nature Chemical Biology 5, 502-507, with a few modifications.
1. The cx6 library of phage from Heinis et al., 2009 Nature Chemical Biology 5, 502-507, (which had been prepared from TG1 cells) was used to infect the non-suppressor strain HB2151 (Carter, Bedouelle & Winter. 1985. Nucleic Acids Res. 13:4431-43), and the infected cells plated. The bacteria were scraped from the plates in about 8 ml 2xTY medium, 30 ug/ml chloramphenicol, 10% glycerol (v/v).
2. About 0.8 ml of the stock was added to 800 ml 2xTY medium with 30 ug/ml chloramphenicol to obtain an OD of about 0.1 at 600 nm. The culture was incubated at 30C, and shaken in a 2 litre flask at 200 rpm for 16hrs.
3. The cell culture was centrifuged at 4,000 rpm (Heraeus Megafuge 2R) for 30 min at 4C. The supernatant was transferred to 200 ml cold 20% PEG, 2.5 M NaCL. The mixture was left on ice for 1 hr.
4. The precipitated supernatant/phage mixture was spun down for 30 min at 4C and the supernatant was discarded.
5. The phage was resuspended in 35 ml PBS, 5mM EDTA followed by spinning for 15 min at 4000 rpm (Heraeus Megafuge 2R) to remove cellular debris. The supernatant was transferred into a new 50 ml Falcon tube.

### Modification of phage with TBMB

1. 5ml of 8mM TCEP (in H₂O) was added to the phage to obtain a final concentration 1mM TCEP. The tube was inverted several time to mix and incubated for 1hr at 42°C water bath.
2. The TCEP was removed by a second PEG precipitation. 10 ml of 20% PEG, 2.5 M NaCl (degassed solution) was added, mixed, and incubated on ice for 45 min and spun for 30 min at 4000 rpm, 4°C.
3. The supernatant was carefully removed and pellet resuspended in 12 ml PBS, 5mM EDTA, 10 uM TCEP (degassed buffer)
4. 3ml of 50 uM TBMB in acetonitrile was added to the 12 ml of reduced phage to obtain a final TBMB concentration of 10 uM. The tube was inverted several times and left at 30 °C for 1 hr in a water bath. The phage were cooled on ice and precipitated with 1/5 volume of 20% PEG, 2.5 M NaCL for 30 min. The phage were collected by spinning at 4000 rpm (Hereaus Megafuge 2R) for 20 min. Supernatant was removed and the phage resuspended in 4 ml of PBS. Phage was transferred into the 2ml Eppendorf tubes and spun at 13000 rpm (Eppendorf benchtop centrifuge) for 10 min. Supernatant was transferred into a new Eppendorf tube and phage infectivity was measured.

### Phage selection: general protocol

### First round of selection

1. Purified and chemically conjugated phage as above was selected against biotinylated MDM2 (bio-MDM2) peptide (res 2-125) immobilized on the surface of the streptavidin-coated Dynabeads (Dynal Biotech). 80 µl of beads were first washed and blocked with 2 % (w/v) Marvell milk powder in PBS (PBSM) for 40 min followed by incubation with 100 nM bio-MDM2 for 20 min in a total volume of 1 ml.
2. Chemically modified phage (10¹⁰-10¹¹TU) was incubated with PBSM for 40 min.
3. Blocked Ag-coated beads from step 1 were washed from the excess of the Ag with 0.1% Tween in PBS (PBST) and incubated with the blocked phage for 30 min in a total volume of 1 ml.
4. Unbound phage were washed with 10x with PBST followed by 2x with PBS. After each third washing step the phage coated beads were transferred into a new Eppendorf tube.
5. Phage were eluted by incubating with 500 µl of 50 mM glycine pH 2.2 for 10 min on a rotating wheel. Eluted phage were neutralized with 250 µl of 1M Tris, pH7.5.
6. 375 µl of phage was incubated with 10 ml of HB2151 cells for 90 min at 37°C without shaking.
7. The infected cells were then shaken for 30 min at 37°C and then plated on a chloramphenicol plate (20x20 cm).
8. The colonies were scraped off the plate in 2xTY, chloramphenicol, 10% glycerol as described above, and stored as a glycerol stock at -80°C. A fraction of the cells was used to prepare phage for the second round of selection.

### Second round of selection

The second round of selection was similar to the first one except for a few modifications.
1. Neutravidin-coated magnetic beads were used instead of streptavidin ones.
2. The amount of antigen used in the selection was 20 nM.
3. Chemically modified phage (10¹⁰-5x10¹⁰ TU) was first treated with 50 ug/ml of chymotrypsin for 2 min followed by blocking with PBSM for 40 min.
4. Unbound phage was washed with 15x with PBST followed by 2x with PBS, otherwise as above.

### Phage selection: variant protocol

Clone 48 was selected using the general protocol as above, whereas clone 10 was developed as a result of a modified protocol being introduced. The modifications are the following:
1. In the first round chemically modified phage were pre-treated with 50 ug/ml of chymotrypsin for 2 min followed by blocking with PBSM for 40 min.
2. In the second round chemically modified phage were first reduced with 5mM DTT for 20 min followed by incubation with 50 µg/ml of chymotrypsin for 2 min and blocking with PBSM for 40 min.

### Peptide synthesis

The encoded peptides from phage clone 48 and phage clone 10 were synthesized with free N- and C-termini. PEP10: H-Ser-Cys-Glu-Leu-Trp-Asn-Pro-Lys-Cys-Arg-Leu-Ser-Pro-Phe-Glu-Cys-Lys-Gly-OH; PEP48: H-Ser-Cys-Val-Arg-Phe-Gly-Trp-Thr-Cys-Asp-Asn-Ser-Trp-His-Gly-Cys-Lys-Gly- OH.

The syntheses was performed by Fmoc-peptide synthesis on a CEM Liberty microwave peptide synthesizer on 0.1mmol Fmoc-Gly-PEG PS resin using a 5-fold excess of Fmoc-amino-acids activated with PyBop in DMF and DIPEA in NMP (1 equivalent and 2 equivalents respectively. Side-chain protecting groups were as follows: Arg(Pbf); Asn(Trt); Asp(OtBu); Cys(Trt); Glu(OtBu); Lys(Boc); Ser(tBu); Thr(tBu); Trp(Boc). Fmoc-deprotection was carried out using 20%v/v Piperidine/DMF containing 0.1M HOBt. The H-peptidyl-resins were washed with DMF, then propan-2-ol and dried *in vacuo.* Cleavage of side-chain protecting groups and from the support was effected using 94:2.5:2.5:1 v/v/v/v TFA/EDT/H₂O/iPr₃SiH for 2 hours. The peptide/TFA mixture was filtered to remove the support and the peptide/TFA mixture was diluted with water and washed with Et₂O (5-times) and the aqueous layer lyophilized.

Reverse-phase hplc were performed on a Phenomenex Jupiter 5µ C18 300Å 250x4.6mm column. Buffer A: 0.1% TFA/H₂0; Buffer B: CH₃CN containing 10% Buffer A. The column was eluted isocratically with 10% Buffer B for 2 minutes, then with a linear gradient of 10-90% over 25 minutes. Detection was at 215/230nm; flow rate of 1.5ml/min.

The peptides were lyophilized and checked by mass spectrometry. PEP10 MALDI-TOF mass (M+H): 2099.9Da (Theory: 2098.4Da.) PEP48 MALDI-TOF Mass (M+H): 2043.8Da (Theory: 2042.8Da.). The peptides were conjugated with TBMB as described in Example 2.

### Binding assays

### Phage ELISA assay

0.6 µg/mL of biotinylated MDM2 peptide (res 2-125) was immobilized on a streptavidin-coated plate (Roche). Plate was blocked with PBSM (but 4% in milk powder) and linear or TBMB-conjugated phage (10⁷ TU/well in PBSM in the presence or absence of 5 mM DTT) was incubated on the plate for 50 min at room temperature. Similarly, phage was first reduced in 5 mM DTT for 20 min, treated with chymotrypsin (50 ug/ml in PBS) for 2 min, mixed with PBSM (final concentration) and incubated on the plate for 50 min at room temperature. Phage was detected using an anti-M13-HRP monoclonal antibody (1:5000, Amersham).

### Fluorescence anisotropy measurements

Titration experiments were run on a Horiba Jobin Yvon fluorimeter equipped with the Hamilton Microlab titrator controlled by laboratory software. The λₑₓ and λₑₘ used were 295nm and 350 nm, respectively. The slit widths for excitation and emission were 5 nm and 15 nm and the integration time 10 s was used for each measurement. The intrinsic fluorescence of tryptophan in peptides 10, 48 was used to measure their binding affinity for MDM2 (res 2-125). The experiments were performed at 23C° in PBS, 5 mM DTT. Usually 250 µl of MDM2 (150 µM) was titrated into 1.2 ml of peptide (1uM). Titration data were analyzed with a standard 1:1 binding model by using the quadratic solution to the equilibrium Kd=[A][B]/[AB]. Kd is the dissociation rate, and [A] and [B] refer to the concentration of a titrant (MDM2) and fluorescent peptides 10 and 48, respectively. The fitting equation contained an extra term to account for linear drift.

The results (Fig. 6 and below) indicate that the affinity of each peptide is sub-micromolar, and in the range 250-750 nM. The measurements for PEP48 were repeated.

PEP10+MDM2, measured λex=295nm, Kd=267nM;
PEP48+MDM2, measured λex=280nm, Kd=760nM;
PEP48+MDM2, measured λex=295nm, Kd=567nM

### Competition assays

The binding of PEP48 phage to MDM2 was competed by a peptide pMI (TSFAEYWNLLSP) that binds to MDM2 at the p53 site with a Kd = 3.3 nM (Pazgier et. al., 2009 PNAS, 106, 4665-4670). 0.6 µg/ml of biotinylated MDM2 peptide (res 2-125) was immobilized on a streptavidin-coated plate (Roche). Plate was blocked with PBSM. TBMB-conjugated phage (10⁷ TU/well in 1% PBSM) was premixed with a range of concentrations of pDI (from 6.94 nM to 1 µM) and incubated on the plate for 75 min at room temperature. Phage was detected using an anti-M13-HRP monoclonal antibody (1:5000, Amersham). The binding of PEP48 phage to MDM2 was inhibited by addition of the pMI peptide, with an estimated IC50 =125 nM.

### Example 2: Enzymatic Cyclisation

### Methodology

### Peptide synthesis

Peptides 1-6 and PK15 with free N termini and amidated C termini were synthesised at a 0.05 mmol scale with standard Fmoc chemistry on an automated peptide synthesizer (Advanced ChemTech 348Ω). After cleavage from the Rink-4-(2',4'-dimethoxyphenyl-Fmoc-aminomethyl)-phenoxy resin with trifluoroacetic acid (95%), triisopropylsilane (2.5%) and H₂O (2.5%), the peptides were precipitated two times in chilled diethylether and the dried peptides were further purified by reversed-phase high-performance liquid chromatography (HPLC) on a Vydac 218TP1022 C18 column (22x250 mm) (Hesperia, USA) with a gradient elution with a mobile phase composed of ACN and 0.1% aqueous trifluoroacetic acid solution at a flow rate of 20 ml/min. The peptides were lyophilized and dissolved in 100 mM Tris-HCI, pH 7.4 and 100 mM NaCl. Peptides 7 and 8 with free N termini and amidated C termini were synthesised on a 25 mg scale by solid-phase chemistry (JPT Peptide Technologies GmbH, Berlin, Germany).

### Peptide cyclisation

Peptides 1-6 (1 µM - 1 mM) in 10 mM Tris-HCI, pH 7.4 and 10 mM NaCl were tested for cyclisation by incubation with microbial transglutaminase of *Streptomyces mobaraensis* (38 kDa) (Zedira, Darmstadt, Germany) (30 nM - 6 µM) for 5 hours at RT. Peptides 7 and 8 were reacted with tris-(bromomethyl)benzene (TBMB) by incubation of 800 µM peptide with 1 mM TBMB in 5 mL 66.5% (v/v) NH₄HCO₃ (100 mM, pH 8) and 28.5% acetonitrile and 5% dimethylsulfoxide (DMSO) for 1 hour at 30°C and purified by HPLC. The HPLC-purified bicyclic peptides 7 and 8 (150 µM) in 10 mM Tris-HCI, pH 7.4 and 10 mM NaCl were incubated with MTGase (1.5 µM) for 6 hours at RT. The peptide PK15 was conjugated with TBMB as described previously (Heinis, C., Rutherford, T., Freund, S. & Winter, G. Phage-encoded combinatorial chemical libraries based on bicyclic peptides. Nat Chem Biol (2009)).

### Mass spectrometric analysis of peptides

The mass of peptides was determined by MALDI-TOF mass spectrometry (Axima-CFR plus, Kratos, Manchester, UK) as follows. Peptides in 0.1% TFA/10-30% acetonitrile in water or MTGase reaction buffer (10 mM Tris-HCI, pH 7.4 and 10 mM NaCl) were mixed with the same volume of matrix solution (10 mg α-cyano-4-hydroxycinnamic acid (a-CHCA) in 1 ml of 50% acetonitrile/50% H₂O/0.1% TFA) and mass measurements were performed. The extent of conversion of the peptides 3 and 4 into the cyclic products was quantified by LC-MS (micromass ZQ 4000, Waters, Milford, USA). The reactions (20 µl) were mixed with 80 µl of 0.1% formic acid and 0.02% TFA in water and 50 µl was separated by reversed phase chromatography on a Vydac 218TP54 C18 column (4.6x250 mm) (Hesperia, USA) and subjected to mass analysis. The quantification was performed by extraction of the ion chromatogram (EIC) and by integration of the peaks. The results were confirmed by MALDI-TOF mass spectrometry (Greis, K.D. et al. MALDI-TOF MS as a label-free approach to rapid inhibitor screening. J Am Soc Mass Spectrom 17, 815-822 (2006)). The deamidation of glutamine to glutamate residue in peptide 6 was analysed by tandem mass spectrometry (MS-MS) (micromass Q-Tof Ultima™, Waters, Milford, USA).

### Treatment of BSA with MTGase

5 µg of Bovine Serum Albumin (68 kDa) purchased from Applichem (Darmstadt, Germany) in 20 µl of MTGase reaction buffer (corresponding to a BSA concentration of 3.7 µM) was incubated with MTGase (concentrations ranging from 60 nM to 6 µM) for 5 hours at RT. Samples were analysed by SDS-PAGE.

### Activity assays

The inhibitory activities (IC₅₀) of the bicyclic and tricyclic peptides 7 and 8 as well as the bicyclic peptide PK15 were determined by incubating human plasma kallikrein (0.1 nM; Innovative Research, Southfiled, USA) in 10 mM Tris-CI, pH 7.4, 150 mM NaCl, 10 mM MgCl₂, 1 mM CaCl₂, 0.1% BSA, 0.01% triton X-100 and 5% DMSO with the peptides at different concentrations (ranging from 2.5 µM to 0.1 nM) and measuring the residual activities of plasma kallikrein with the fluorogenic substrate Z-Phe-Arg-AMC (100 µM) at RT. Fluorescence intensities were recorded for 1 hour with excitation and emission wavelengths of 355 nm and 460 nm on a fluorescence plate reader (Molecular Devices, Sunnyvale, USA).

### Aminopeptidase treatment of peptides

Peptide 7 before (300 µM) and after (75 µM) MTGase treatment in reaction buffer was incubated with different amounts of leucyl aminopeptidase from *Aeromonas proteolytica* (EC 3.4.11.10; Sigma, A8200) (3 to 352 ng, corresponding to concentrations of about 50 nM to 6 µM) in a final volume of 2 µl for 30 min at RT. The mass of the peptides was determined by MALDI-TOF mass spectrometry.

### Quantitative cyclisation of peptides with a microbial transglutaminase

We used the 38 kDa microbial transglutaminase (MTGase) of *Streptomyces mobaraensis* to test the enzymatic cyclisation of peptides (Yokoyama, K., Nio, N. & Kikuchi, Y. Properties and applications of microbial transglutaminase. Appl Microbiol Biotechnol 64, 447-454 (2004)). The MTGase was reported to be relatively stable in food processing and other applications and in contrast to mammalian TGases it is active in the absence of Ca²⁺. Phage selections with random heptapeptide libraries had revealed that MTGase of *Streptomyces mobaraensis* accepts a broad range of substrates wherein certain peptides are preferred as glutamine-donor substrates (Sugimura, Y., Yokoyama, K., Nio, N., Maki, M. & Hitomi, K. Identification of preferred substrate sequences of microbial transglutaminase from Streptomyces mobaraensis using a phage-displayed peptide library. Arch Biochem Biophys 477, 379-383 (2008)). We designed a peptide with the MTGase substrate sequence WALQRPH (the glutamine-donor residue is underlined; Sugimura et al., 2008), a flexible 3-amino acid spacer (GGG) and a lysine acceptor-residue (peptide 1, H-WALQRPHGGGKS-NH₂; **Table 1).** We chose to position the glutamine-donor substrate peptide (WALQRPH) at the N-terminus and the lysine residue at the C-terminus of the peptide because a model of a peptide-MTGase complex suggested that only in this configuration, the peptide linker could bend back to bring the lysine and glutamine side chains into close proximity. Incubation of peptide 1 (3.8 µM) with MTGase (30 nM) and mass spectrometric analysis revealed a single product with a 17 Da smaller mass suggesting that an ammonium molecule was eliminated and the peptide was cyclised **(****Figure 1B**). At higher peptide concentrations, formation of dimers was observed.

### Substrate specificity of MTGase in cyclisation reactions

In order to determine the minimal amino acid sequence that is accepted by MTGase as a substrate in a cyclisation reaction, we synthesised a range of peptides in which the MTGase glutamine-donor substrate sequence WALQRPH was truncated **(Table 1)** and measured the extent of cyclisation upon incubation with MTGase. While a peptide without the three N-terminal amino acids Trp-Ala-Leu (peptide 2; H-GQRPHGGGKS-NH₂) was not cyclised at all, the substitution of the three amino acids Arg-Pro-His with Ser-Gly-Ser residues yielded a substrate (peptide 3, H-WALQSGSGGGKS-NH₂) that was cyclised quantitatively. This result was pleasing since it suggested that cyclic peptides with variable sequences in the ring can be generated in MTGase catalysed reactions. Experiments with peptides truncated partially at the N-termini revealed that a peptide with Ala-Leu at the N-terminal side of the glutamine residue is cyclised (peptide 4, H-ALQSGSGGGKS-NH₂) but not a shorter peptide (peptide 5, H-LQSGSGGGKS-NH₂) **(Table 1).** The relatively small sequence requirement of MTGase determined in this set of experiments should allow the generation of cyclic peptides that are not much compromised in their design.

### Catalytic activity of MTGase in cyclisation reactions

In order to quantify the catalytic activity of MTGase in cyclisation reactions we incubated the two substrate peptides 3 (H-WALQSGSGGGKS-NH₂) and 4 (H-ALQSGSGGGKS-NH₂) (90 µM) with different enzyme concentrations (1 nM to 6 µM) and quantified the extent of cyclisation by LC-MS and MALDI-TOF MS²² **(****Figure 2A****).** After incubation at room temperature for 5 hours, the peptides 3 and 4 were quantitatively cyclised at MTGase concentrations of 60 nM and 1.5 µM, respectively. Peptide 4 having a shorter glutamine-donor substrate sequence was cyclised by MTGase around 5-times slower than peptide 3. To assess the non-specific ligation of glutamine and lysine residues we incubated 5 µg of bovine serum albumin (BSA) with the same concentrations of MTGase (from 60 nM to 6 µM) and analysed the extent of protein cross-linking by SDS PAGE. Although BSA has 7 and 30 solvent accessible glutamine and lysine residues corresponding to concentrations as high as 28 and 120 □M, no unspecific cross-linkage of protein was observed **(****Figure 2B****),** suggesting that peptide can also be cyclised in the presence of proteins or even mixtures of proteins (e.g. in the cytoplasm of a mammalian or bacterial cell).

### MTGase catalysed deamidation of glutamine

In a control experiment we had incubated a peptide without a lysine-acceptor residue at the C-terminus (peptide 6, H-WALQSGSGGGGS-NH₂; **Table 1)** (28 to 280 µM) with MTGase (6 µM) expecting that it is not cyclised. Unexpectedly, MALDI-TOF analysis showed reproducibly a product with a mass of 1062 Da which is 1 dalton larger than the mass of the peptide 6 (1061 Da) before MTGase treatment **(****Figure 3B****).** Such a mass shift is expected for a deamidation reaction in which the glutamine residue of peptide 6 is attacked by the active site thiol of MTGase and the intermediate is hydrolyzed and a glutamate residue is formed **(****Figure 3A****).** The transformation of glutamine into glutamate was confirmed by sequencing of the reaction product using tandem mass spectrometry (results not shown). Deamidation of the glutamine residues in the peptides 1-5 was not detected suggesting that cyclisation through transamidation is much faster and that deamidation happens only if no primary amine is available.

### Generation of tricyclic peptides

Next we tested whether a chemical cyclisation reaction that was previously used to generate bicyclic peptides (Heinis, C., Rutherford, T., Freund, S. & Winter, G. Phage-encoded combinatorial chemical libraries based on bicyclic peptides. Nat Chem Biol (2009); Timmerman, P., Beld, J., Puijk, W.C. & Meloen, R.H. Rapid and quantitative cyclization of multiple peptide loops onto synthetic scaffolds for structural mimicry of protein surfaces. Chembiochem 6, 821-824 (2005)), can be combined with the enzymatic cyclisation to obtain a tricyclic peptide **(****Figure 4A****).** We synthesised the bicyclic peptide inhibitor of human plasma kallikrein PK15 (Heinis et al., 2009) with the exocyclic peptide appendices Ala-Leu-Gln-Ala at the N-termini and Ala-Lys-Ser at the C-termini by reacting the linear peptide 7 (H-ALQACSDRFRNCPADEALCAKS-NH₂; **Table 1)** via the three cysteine side chains to the small organic compound tris-(bromomethyl)benzene. Incubation of the bicyclic peptide with MTGase yielded a single product with a mass of 2464 Da which is 17 daltons smaller than the bicyclic peptide suggesting that the two ends were linked in a transamidation reaction **(****Figures 4B and 4C****).** An identical experiment with a peptide having an additional tryptophane residue at the N-terminus (peptide 8, H-WALQACSDRFRNCPADEALCAKS-NH₂; **Table 1)** gave an equivalent result. To confirm the cyclisation of the third loop, tricyclic peptide 7 was treated with the leucyl aminopeptidase from *Aeromonas proteolytica* (EC 3.4.11.10) which cleaves amino acids from the N-terminus of well accessible peptide chains. Mass spectrometric analysis of the reaction products showed that the unconjugated glutamine residue in the bicyclic peptide was degraded together with the other exocyclic amino acids (Ala-Leu-Gln-Ala) if 70 ng or more exopeptidase was added **(****Figure 5A****).** In contrast, higher exopeptidease concentrations were required to degrade the Gin-Ala dipeptide in the tricyclic peptide where the glutamine side chain is linked to lysine **(****Figure 5B****).** Together with the observed mass change of 17 dalton upon incubation of the bicyclic peptide with MTGase, these results supported the tricyclic peptide configuration shown in **Figure 4B****.** The tricyclic peptides 7 and 8 inhibited human plasma kallikrein about 70-fold weaker (IC₅₀ of 200 nM and 196 nM) than the parental inhibitor PK15 (IC₅₀ = 3 nM) **(Table 2).** Measurement of the activities of the peptides 7 and 8 as bicyclic structures (before cyclisation with MTGase; IC₅₀ of 259 nM and 185 nM) revealed that the activity drop is due to the peptide appendices and not because of the formation of the third peptide loop **(Table 2).** This result is in line with the finding that C-terminal conjugation of PK15 to a protein reduces its activity by a factor of around 10 (Heinis et al., 2009).

### Tables

**Table 1**

| Name | Amino acid sequence | MTGase cyclisation | Mass (Da) |
|---|---|---|---|
| peptide 1 | H-*WAL*Q*RPH*GGGKS-NH₂ | Yes | 1291.68 |
| peptide 2 | H-GQ*RPH*GGGKS-NH₂ | No | 978.50 |
| peptide 3 | H-*WAL*QSGSGGGKS-NH₂ | Yes | 1132.55 |
| peptide 4 | H-*AL*QSGSGGGKS-NH₂ | Yes | 946.47 |
| peptide 5 | H-*L*QSGSGGGKS-NH₂ | No | 875.44 |
| peptide 6 | H-*WAL*QSGSGGGGS-NH₂ | No | 1061.48 |
| peptide 7 | H-*AL*QACSDRFRNCPADEALCAKS-NH₂ | Yes | 2481.08 |
| peptide 8 | H-*WAL*QACSDRFRNCPADEALCAKS-NH₂ | Yes | 2667.16 |

**Table 2**

| Name | Amino acid sequence | IC₅₀ (nM) | |
|---|---|---|---|
| | | Before MTGase cyclisation (bicyclic) | After MTGase cyclisation (tricyclic) |
| PK15 | H-ACSDRFRNCPADEALCG-NH₂ | 3 | - |
| peptide 7 | H-ALQACSDRFRNCPADEALCAKS-NH₂ | 259 | 200 |
| peptide 8 | H-*WAL*QACSDRFRNCPADEALCAKS-NH₂ | 185 | 196 |

**Table 2.** Inhibition of human plasma kallikrein with bicyclic and tricyclic peptides. Indicated are the amino acid sequences of PK15,⁴ peptide 7 and 8 and their inhibitory activities before (as bicyclic TBMB conjugates) and after MTGase treatment (tricyclic peptides).

## Claims

1. A repertoire of peptide ligands which is displayed using a genetic display system, wherein each peptide ligand comprises a polypeptide variant linked to a molecular scaffold at n attachment points, wherein said polypeptide is cyclised and forms n separate loops subtended between said n attachment points on the molecular scaffold, wherein n separate loops = n attachment points and n is 2 or 3, and said molecular scaffold possesses n scaffold reactive groups and n-fold molecular symmetry.

2. A repertoire according to claim 1, wherein the polypeptide is cyclised after attachment to the molecular scaffold.

3. A repertoire according to any preceding claim, wherein the polypeptide is cyclised by enzymatic means, for example wherein the enzyme is a transglutaminase, for example *Streptomyces mobaraensis* transglutaminase.

4. A repertoire according to any preceding claim which is capable of binding to more than one separate target.

5. A repertoire according to claim 4, wherein the loop formed by polypeptide cyclisation binds to a target which is different to that bound by at least one other loop.

## Patentansprüche

1. Verzeichnis von Peptidliganden, das unter Verwendung eines genetischen Anzeigesystems angezeigt wird, wobei jeder Peptidligand eine Polypeptidvariante umfasst, die mit einem molekularen Gerüst an n Befestigungspunkten verlinkt ist, wobei das Polypeptid zyklisiert ist und n getrennte Schleifen bildet, die zwischen den n Befestigungspunkten an dem molekularen Gerüst aufgespannt sind, wobei n getrennte Schleifen = n Befestigungspunkte sind und n 2 oder 3 ist, und das molekulare Gerüst n reaktive Gerüstgruppen und eine n-fache molekulare Symmetrie besitzt.

2. Verzeichnis nach Anspruch 1, wobei das Polypeptid nach dem Befestigen an dem molekularen Gerüst zyklisiert wird.

3. Verzeichnis nach einem vorstehenden Anspruch, wobei das Polypeptid durch enzymatische Mittel zyklisiert wird, wobei das Enzym eine Transglutaminase, beispielsweise eine *Streptomyces mobaraensis* Transglutaminase ist.

4. Verzeichnis nach einem vorstehenden Anspruch, das imstande ist, sich an mehr als ein getrenntes Ziel zu binden.

5. Verzeichnis nach Anspruch 4, wobei sich die durch Polypeptid-Zyklisierung gebildete Schleife an ein Ziel bindet, das sich von jenem unterscheidet, das durch mindestens eine andere Schleife gebunden wird.

## Revendications

1. Répertoire de ligands peptidiques qui est présenté en utilisant un système de présentation génétique, dans lequel chaque ligand peptidique comprend un variant polypeptidique lié à un échafaudage moléculaire à n points de fixation, dans lequel ledit polypeptide est cyclisé et forme n boucles séparées sous-tendues entre lesdits n points de fixation sur l'échafaudage moléculaire, dans lequel n boucles séparées = n points de fixation et n est 2 ou 3, et ledit échafaudage moléculaire possède n groupes réactifs d'échafaudage et une symétrie moléculaire d'ordre n.

2. Répertoire selon la revendication 1, dans lequel le polypeptide est cyclisé après fixation à l'échafaudage moléculaire.

3. Répertoire selon une quelconque revendication précédente, dans lequel le polypeptide est cyclisé par un moyen enzymatique, par exemple dans lequel l'enzyme est une transglutaminase, par exemple une transglutaminase de *Streptomyces mobaraensis.*

4. Répertoire selon une quelconque revendication précédente qui est capable de se lier à plus d'une cible séparée.

5. Répertoire selon la revendication 4, dans lequel la boucle formée par cyclisation polypeptidique se lie à une cible qui est différente de celle liée par au moins une autre boucle.
